# EUROPEAN PATENT SPECIFICATION

(11) **EP 4 221 517 B1**
(45) Date of publication and mention of the grant of the patent: **25.09.2024**
(21) Application number: 21721931.0
(22) Date of filing: 29.04.2021
(51) Int. Cl.: A61K 31/702, A61K 31/7004, A61K 35/744, A61K 36/48, C12P 7/56, C12P 19/02, A23J 1/14, A23J 3/34, A23K 10/12, A23K 20/163, A23K 50/60, A23L 7/104, A23L 7/10, A23L 25/00, A23L 33/105, A23L 33/21, C12N 9/14, C12N 9/42, A61K 36/06, C12P 19/14

(54) **FEED OR FOOD INGREDIENT DERIVED FROM FIBRE-RICH BIOMASS OF SOY HULLS**
AUS FASERREICHER BIOMASSE GEWONNENER FUTTER- ODER LEBENSMITTELBESTANDTEIL
ALIMENTATION OU INGRÉDIENT ALIMENTAIRE DÉRIVÉS DE BIOMASSES RICHES EN FIBRES

(30) Priority: 02.10.2020 EP 20199889
(43) Date of publication of application: 09.08.2023
(73) Proprietor: Hamlet Protein A/S, 8700 Horsens (DK)
(72) Inventor: THIRUP, Laila, 8700 Horsens (DK); BRØKNER, Christine, 8700 Horsens (DK); DICKOW, Jonatan A., 8700 Horsens (DK); RASMUSSEN, Pernille Toft, 8700 Horsens (DK)
(74) Representative: Zacco Denmark A/S
(86) International application number: PCT/EP2021/061281
(87) International publication number: WO 2022/069084

(56) References cited:
- WO-A2-2013/171259
- CN-A- 105 230 701
- CN-A- 105 876 082
- CN-A- 110 527 703
- CN-A- 111 109 465
- US-A1- 2009 098 638
- US-A1- 2012 282 239
- US-B2- 10 428 355
- DATABASE WPI Week 201678, Derwent World Patents Index; AN 2016-58089S
- DATABASE WPI Week 202004, Derwent World Patents Index; AN 2020-42174B, XP002802133
- DATABASE WPI Week 2020, Derwent World Patents Index; AN 2019-A2962Q

## Description

### FIELD OF THE INVENTION

The present invention relates to a feed or food ingredient derived from a fibre-rich plant biomass of soy hulls, a method for the product of the feed/food ingredient, a use of the feed/food ingredient, and a feed or food product comprising the feed/food ingredient.

### BACKGROUND OF THE INVENTION

Fibre is increasingly important in alternative feeding of production animals. Previously, fibre has been regarded as an anti-nutritional factor that reduces the energy concentration and digestibility of feed, leading to poorer animal performance. Increasing evidence during the last two to three decades has, however, shown that a certain fibre inclusion is beneficial for performance and development of a healthy gut of the animal; Agyekum & Nyachoti (2017), Molist et al (2009). Both the content of soluble and insoluble non-starch polysaccharides (NSP) as well as the content of prebiotic oligosaccharides are part of the dietary fibre which can have positive effects in the animal. More generally, fibre is now investigated intensively to be one of the solutions to restrictions in use of antibiotics and zinc in the animal sector. With less use of antibiotics and zinc, bad performance, disease, and diarrhoea will increase, and it is a serious problem.

Dedicated fibre sources are increasingly used in monogastric feeding. Soy hulls is such a dedicated fibre source, high in total and in insoluble fibres. Surprisingly, it has now been found by the present inventors, that when one treats the fibre biomass soy hulls with specific carbohydrate targeting enzymes, the fibres can release a rather large amount of oligosaccharides, compared to many other natural biomasses that are high in fibre content. Depending on the type of targeting enzymes applied, the nature of oligosaccharides released from the soy hulls and the other biomasses can be different.

In some animal feeding, it is now common to add "exogenous" enzymes to the final diet formulation, but the enzyme is not in action before it is consumed with the rest of the diet, working in the gut environment of the animal (Kiarie et al 2013, Scapini et al 2018). The use of carbohydrase enzymes as a production aid to increase the prebiotic oligosaccharide content of feed biomasses has only been described to a limited degree. The potential effect of some oligosaccharides is expected to be prebiotic, when the oligosaccharide-enriched source is fed to production animals, as for example pigs and poultry, resulting in increased animal performance and health. For human food products, or research purposes, biomasses have been treated with enzymes and/or physical/chemical means to produce, extract and purify prebiotic oligosaccharides. Alternatively, some prebiotic products are chemically synthesized and purified (for example polydextrose, dextrin) for the same use. This type of products are expensive and, therefore, not used to a significant degree in animal feed; Babber et al (2015); Aachary & Prapulla (2011); Dotsenko et al (2017); Kurakake et al (2006).

US 2009/098638 A1 relates to a method for increasing the amount of glucose and other sugars and peptides by the hydrolysis of various fibre feedstocks, including e.g. soy hulls, with a protease and an enzyme selected from cellulase and hemicellulase. The method provides a product with high glucose content.

WO2013/171259 A2 relates to a method for the production of a solid bio-product from a biomass wherein at least 80 % of the original indigestible oligosaccharide content has been degraded by reaction with an enzyme preparation containing α-galactosidase. Soy hulls are not disclosed for the method.

CN 111 109 465 A relates to a method for preparing biological feed for pigs by processing a mixed raw material/biomass as a dry powder with a fermentation broth comprising a mixture of enzymes and bacteria. Soy hulls are not disclosed for the method.

At present, it appears that there are no feed or food products on the market originating from soy hulls by carbohydrase enzyme treatment, having increased specialised oligosaccharide content in comparison with the untreated soy hulls. There is neither any prior art that describes a carbohydrase enzyme treated soy hull for consume; some literature only describe that soy hulls can be added to the diet together with an external carbohydrase enzyme source before feeding of the animal.

The present inventors have tested different soy hulls products treated with carbohydrate targeting enzymes, such as mannanase, xylanase, or pectinase, in piglet feeding trials and in vitro piglet trials, and found improved performance compared to a control group.

The object of the present invention is to provide a new feed or food ingredient having a distinctive composition, that originates from a fibre-rich plant biomass of soy hulls.

Another object is to provide, on a favourable cost basis for the applied carbohydrate targeting enzymes, a new improved feed or food ingredient originating from soy hulls, which source comprises both a certain amount of complex carbohydrates in the form of dietary fibres and an increased amount of prebiotic oligosaccharides derived by action of carbohydrate targeting enzymes, which is advantageous when used in an animal or human diet.

Yet another object is to provide a new feed or food ingredient obtainable by treatment of soy hulls with carbohydrate targeting enzymes.

Furthermore, it is an object to provide an improved feed or food ingredient containing prebiotic oligosaccharides, having higher digestibility in comparison with the untreated (raw) soy hulls.

Finally, it is an object to provide a new product with high water binding capacity and low viscosity; both these properties are important for the product's application in an animal diet.

These objects are fulfilled with the product of the present invention.

### SUMMARY OF THE INVENTION

Accordingly, in a first aspect the present invention relates to a feed or food ingredient derived from soy hulls, wherein the feed or food ingredient comprises dietary fibres from soy hulls in the form of soluble and insoluble polysaccharides, and wherein part of the dietary fibres from the soy hulls have been degraded by one or more carbohydrase(s) selected from mannanase(s), pectinase(s), xylanase(s), glucanase(s), and cellulase(s) into oligosaccharides having from 3 to 30 monomer sugar units (Oligosaccharides DP 3-30) to provide the feed or food ingredient comprising 4 % by weight or more of said Oligosaccharides DP 3-30, calculated as not including the soy oligosaccharides raffinose, stachyose, and verbascose, wherein the feed or food ingredient further comprises yeast, and with the proviso that the feed or food product does not comprise more than 5 % by weight monosaccharides.

In the context of the present invention, the oligosaccharides DP 3-30 derived from the dietary fibres do not comprise raffinose, stachyose, and verbascose.

Surprisingly, the present inventors have found that when they treated a range of different fibre-rich plant biomasses with selected carbohydrases, viz. enzymes acting on complex polysaccharides, soy hulls stood out as a biomass from which a quite large amount of oligosaccharides can be released. Also, a number of selected mannanases, pectinases, xylanases, cellulases, and β-glucanases produce a significant amount of oligosaccharides from soy hulls, in comparison with what is typically seen from other biomasses. It is expected that some of these oligosaccharides can act as prebiotics in animal feed or human food, depending on the chemical characteristics of the oligosaccharides.

It is further surprising that such products when they are applied in the animal diet can increase animal performance, such as piglet and poultry performance. The inventors have confirmed this in piglet feeding trials. The known mode of actions lying behind the increased performance when one uses prebiotic oligosaccharides are: improving/stabilizing a healthy microbial community in the gut, reduction of the risk of intestinal infection and inflammation, improvement of intestinal functions (stool bulking, regulatority and consistency), improvement of intestinal barrier function, regulation/modulation of immune functions and modulation of gastro-intestinal peptide production and energy metabolism; Roberfroid et al (2010),. The best known and best studied and documented oligosaccharides are fructo-oligosaccharides (FOS); Ghoddusi et al (2007); Probert et al (2004), typically derived from inulin; and mannan-oligosaccharides (MOS); Corrigan et al (2015); Zivkovic et al (2011); Kim et al (2010). In the case of MOS, it is the type of MOS derived from yeast cell walls that are well documented for their effect. They are α-1,3 and α-1,6 branched mannans. Examples of new up-coming groups of prebiotic oligosaccharides are: xylo-oligosaccharides (XOS); Liu et al (2018); Moura et al (2008); Aachary & Prapulla (2011); Dotsenko et al (2017); Nielsen et al (2014); pectin derived oligosaccharides (POS); Babber et al (2015); Chung et al (2017); Strube et al (2015); and β-glucan derived oligosaccharides; Meyer et al (2015); Miguez et al (2016). Galacto-oligosaccharides (GOS) can be synthesized from lactose, and is also a new prebiotic oligosaccharide; Torres et al (2010). Chemical characteristics important for an oligosaccharide to have prebiotic effects or not, are the type of chemical bonds between sugar moieties, the identity of the sugar molecules, and their branching; Markowiak & Slizewska (2018); Pourabeden & Zhao (2015), Kim et al (2019).

The recommended amounts in an animal feed of MOS to swine is approx. 0.25 to 0.5% of the final feed formulation, and the recommended amounts of FOS is approx. 0.03 to 1.25 % of the feed formulation. Maribo (2005): "Tilsaetningsstoffer til svin. Landsudvalget for svin". This supports the idea that even small amounts of prebiotic oligosaccharides added to a production animal feed make a difference.

In a second aspect, the invention relates to a method for the production of a feed or food ingredient according to the invention, comprising the following steps:
- mixing soy hulls with one or more carbohydrase(s) selected from mannanase(s), pectinase(s), xylanase(s), glucanase(s), and cellulase(s);
- adding yeast to the mixture of soy hulls and carbohydrase(s);
- hydrolysing the mixture under conditions, where the dry matter (DM) content is 55 % by weight or less, in a time period of from 1 to 48 hours, at a temperature from 20 to 60 °C;
- optionally fully inactivating the activity of the one or more carbohydrase(s); and
- isolating the feed or food ingredient.

The invention further provides a feed or food product or a nutritional supplement for production animals containing from 0.5 to 99% by weight of the feed or food ingredient of the invention, such as a feed product suitable for use in a diet for production animals, preferably newborn and young animals, such as piglets, calves, and poultry, in need of prebiotic oligosaccharides. The invention is defined by the appended set of claims.

### Definitions

In the context of the current invention, the following terms are meant to comprise the following, unless defined elsewhere in the description.

The term "comprising" is to be interpreted as specifying the presence of the stated part(s), step(s), feature(s), composition(s), chemical(s), or component(s), but does not exclude the presence of one or more additional parts, steps, features, compositions, chemicals or components. E.g., a composition comprising a chemical compound may thus comprise additional chemical compounds, etc.

### Biomass:

Comprises biological material produced by the photosynthesis and that can be used in industrial production. In the present context, biomass refers to fibre-rich plant matter in the form of soy hulls. The soy hulls may be applicable in raw form or in a disintegrated and/or pretreated form, depending of its nature and of the skilled person's approach to a specific raw material.

### Soya bean products:

Refers to plant matter in the form of soya bean products, in the present context in particular products from soy hulls, and mixtures thereof. The soya bean can be from any soya bean source, such as from South or North America or Asia or Europe, and it can be of gene modified origin (GMO) or of non-gene modified origin (non-GMO).

### Soy hulls

Soy hulls are a dedicated fibre source, high in total and in insoluble fibres and also contain some protein. Soy hulls are a by-product of soy bean processing and consist of the soy bean coating. Soy hulls contain complex carbohydrates, such as pectin, hemicelluloses, and cellulose, and is a good source of dietary fibres.

### Disintegrated:

Means, for example, disintegrated by chemical or physical means according to methods known in the art, e.g. milling, blending, cooking, and/or acid or alkaline treatment. The skilled person will know whether disintegration will be necessary and, if required, which disintegration may be appropriate for the specific plant biomass applied in the invention.

### Oligosaccharides:

An oligosaccharide is normally defined as a saccharide oligomer containing a small number (3-10) of component monomer sugars. In the context of the present invention, an oligosaccharide is defined broader, and it can be a saccharide oligomer containing from 3 to 30 monomer sugar units.

### Oligosaccharides 3-30 sugar units/Oliqosaccharides DP 3-30

In the context of the present invention, these terms designate saccharide oligomers containing 3-30 monomer sugar units and is a feature applied in the definition of the present new product. In the context of the present invention, soy oligosaccharides, in particular raffinose, stachyose, and verbascose that are included in the raw soy hulls, are not included in the herein defined DP 3-30.

### Prebiotic oligosaccharides

An oligosaccharide to be regarded as prebiotic must not be hydrolysed or absorbed in the upper part of the gastrointestinal tract, and must be assimilated selectively by beneficial microorganisms in the colon, promoting beneficial luminal or systemic effects; Meyer et al (2015); Miguez et al (2016). They can be fermented by microorganisms in the hindgut, but need to "survive" until they reach there; Smiricky-Tjardes et al (2003).

### Monosaccharides/mono sugars

Monosaccharides, or mono sugars, are simple saccharide monomers, comprising five and/or six carbon atoms, and is the basic units of sugar and carbohydrates. In the context of the present invention, monosaccharides/mono sugars are in particular glucose, fructose, and galactose.

### Polysaccharides

Polysaccharides are saccharide polymers containing a large number of component monomer sugars, also known as complex carbohydrates. Polysaccharides may be soluble and insoluble.

### Dietary fibres

Dietary fibres comprise soluble and insoluble, non-starch polysaccharides and may comprise oligosaccharides, lignin, and resistant starch. The raw biomasses selected in the context of the present invention only contain minor amounts of oligosaccharides and resistant starch, or are substantially free of resistant starch.

### Carbohydrase

Carbohydrase in the present context is any enzyme capable of hydrolysing any carbohydrate structure such as mannanase(s), pectinases(s), xylanase(s), glucanase(s), and cellulase(s).

### Yeast

In the present context, yeast may in particular be selected among *Saccharomyces cerevisiae* strains, including spent brewer's yeast and spent distiller's yeast and spent yeast from wine production, bioethanol yeast, or spent yeast from bioethanol production, baker's yeast, and yeast strains fermenting C5 sugars.

### Microorganisms

Microorganisms are organisms which are microscopic, making them too small to be seen by the unaided human eye. Microorganisms include bacteria, fungi, archaea, protists, and viruses.

### Lactic acid bacteria

Are usually found in decomposing plants and milk products and produce lactic acid as the major metabolic end product of carbohydrate bio-conversion. The lactic acid bacteria are genera of microorganism which produce organic acids, such as lactic acid and some acetic acid, as metabolic products of carbohydrate bio-conversion. The genera are in particular, but are not limited to, *Lactobacillus, Pediococcus, Lactococcus, Enterococcus, Weisella, Streptococcus, and Leuconostoc.*

### Other genera

In the context of the present invention, other genera refer to the most relevant other bacterial genera in relation to the invention. They comprise a number of genera which also produce organic acids, such as lactic acid and acetic acid, as metabolic products of carbohydrate bio-conversion, but often to a lesser extent than the lactic acid bacteria. In the context of the present invention other genera than the lactic acid comprises, but are not limited to, *Bacillus, Bifidobacterium, Brevibacillus, Propionibacterium, Clostridium, and Geobacillus.* Certain strains are used as probiotics.

### Feed products

Comprise ready-to-use feed or feed ingredients for production animals such as piglets, calves, poultry, furred animals, and sheep.

### Food products

Comprise ready-to-use food or food ingredients for human nutrition.

The invention is illustrated in the drawing, wherein:
Figure 1 shows a first screening results after incubation of soy hull biomass with different carbohydrate targeting enzymes under pH regulation. Oligosaccharides DP 3-30 measured do not include raffinose, stachyose, and verbascose.
Figure 2 shows a similar screening after a similar incubation of soy hull biomass without pH regulation, but with baker's yeast added. Oligosaccharides DP 3-30 measured do not include raffinose, stachyose, and verbascose.
Figure 3 shows TLC results of incubation of soy hulls with different carbohydrase enzymes.
Figure 4 shows the effect of hydrolysis time when Vland pectinase or mannanase is used for incubation of the soy hull biomass. Oligosaccharides DP 3-30 measured do not include raffinose, stachyose, and verbascose.
Figure 5 shows the effect of hydrolysis time and enzyme dose when soy hull biomass is incubated with different carbohydrase enzymes. Oligosaccharides DP 3-30 measured do not include raffinose, stachyose, and verbascose.
Figure 6 shows as a comparison quantification of oligosaccharides DP 3-30 (not include raffinose, stachyose, and verbascose), in different biomasses, either the raw biomass, an incubated biomass without enzymes, or enzyme treated biomasses after incubation with carbohydrase enzyme.
Figure 7 shows TLC for a soy hulls biomass after incubation with Depol 793L (pectinase), xylanase, mannanase, and cellulase from Strowin, respectively.
Figure 8 shows water holding in soy hulls in comparison with different biomasses, either in raw form or after treatment according to the invention.
Figure 9 shows viscosity in different biomasses, either in raw form or after treatment according to the invention.

### DETAILED DESCRIPTION OF THE INVENTION

*The product of the invention in its first aspect:*
In its first aspect, the invention relates to a feed or food ingredient derived from soy hulls, wherein the feed or food ingredient comprises dietary fibres from the soy hulls in the form of soluble and insoluble polysaccharides, and wherein part of the dietary fibres from the soy hulls have been degraded by one or more carbohydrase(s) selected from mannanase(s), pectinase(s), xylanase(s), glucanase(s), and cellulase(s) into oligosaccharides having from 3 to 30 monomer sugar units (Oligosaccharides DP 3-30) to provide the feed or food ingredient comprising 4% by weight or more of the Oligosaccharides DP 3-30, calculated as not including the soy oligosaccharides raffinose, stachyose, and verbascose, wherein the feed or food ingredient further comprises yeast, and with the proviso that the feed or food product comprises 5 % by weight monosaccharides or less.

In this context, the oligosaccharides DP 3-30 derived from the dietary fibres do not comprise raffinose, stachyose and verbascose.

In one embodiment of this aspect, part of the dietary fibres from soy hulls have been degraded by the one or more carbohydrase(s) into oligosaccharides providing prebiotic effect.

In any embodiment, the feed or food product comprises 5 % by weight or less monosaccharides; such as 4 % by weight or less; 3 % by weight or less; 2 % by weight or less; 1 % by weight or less; 0.5 % by weight or less; is substantially free of monosaccharides, or is free of monosaccharides.

In another embodiment, the feed or food ingredient may comprise 4 % by weight or more of the oligosaccharides having from 3 to 30 monomer sugar units (Oligosaccharides DP 3-30), such as 5 % by weight or more, 6 % by weight or more, 7 % by weight or more, 8 % by weight or more, 9 % by weight or more, 10 % by weight or more, 12 % by weight or more, 15 % by weight or more, or 20 % by weight or more, of oligosaccharides having from 3 to 30 monomer sugar units (DP3-30). In this context, the oligosaccharides DP 3-30 derived from the dietary fibres do not comprise raffinose, stachyose, and verbascose.

In any of the embodiment of the invention in its first aspect, the feed or food ingredient further comprise live yeast, such as live yeast selected among Saccharomyces cerevisiae strains, including spent brewer's yeast, baker's yeast, spent distiller's yeast, and spent yeast from wine production, bioethanol yeast, or spent yeast from bioethanol production, and yeast strains fermenting C5 sugars. The live yeast may e.g. be present in an amount of from 0.05 to 10 %, such as 0.10%, 0.15%, 0.20%, 0.25%, 0.5%, 1%, 1.5%, 2%, 2.5%, 3%, 3.5%, 4%, 4.5, 5%, 6%, 7%, 8% or 9%.

In any of the embodiment of the invention in its first aspect, the oligosaccharides raffinose, stachyose, and/or verbascose present in the raw soy hulls may sometimes have been fully or partly degraded.

In any of the embodiment of the invention in its first aspect, the feed or food ingredient may sometimes further comprise one or more microorganisms and/or metabolic products of carbohydrate degradation of the soy hulls by one or more microorganisms.

In any of the embodiment of the invention in its first aspect, the feed or food ingredient may sometimes further comprise one or more microorganisms selected from lactic acid bacteria and/or metabolic products of carbohydrate degradation of the soy hulls by lactic acid bacteria, such as *Lactobacillus, Pediococcus, Lactococcus, Enterococcus, Weisella, Streptococcus, and Leuconostoc.*

The feed or food ingredient may sometimes further comprise one or more microorganisms and/or metabolic products of carbohydrate degradation of the soy hulls by the one or more microorganisms selected from *Bacillus, Bifidobacterium, Brevibacillus, Propionibacterium, Clostridium, and Geobacillus.*

The feed or food ingredient may be derived from soy hulls, wherein part of the soy hull fibres have been degraded by one or more β-mannanase(s) into oligosaccharides, comprising 4 % by weight or more of oligosaccharides having from 3 to 30 monomer sugar units, such as 5 % by weight or more, 10 % by weight or more, 15 % by weight or more, or 20 % by weight or more, of oligosaccharides having from 3 to 30 monomer sugar units (DP3-30). In this context, the oligosaccharides DP 3-30 derived from the dietary fibres do not comprise raffinose, stachyose, and verbascose.

The feed or food ingredient may be derived from soy hulls, wherein part of the soy hull fibres have been degraded by one or more pectinase(s) into oligosaccharides, comprising 4 % by weight or more of oligosaccharides having from 3 to 30 monomer sugar units, such as 5 % by weight or more, 10 % by weight or more, 15 % by weight or more, or 20 % by weight or more, of oligosaccharides having from 3 to 30 monomer sugar units (DP3-30). In this context, the oligosaccharides DP 3-30 derived from the dietary fibres do not comprise raffinose, stachyose, and verbascose.

The feed or food ingredient may be derived from soy hulls, wherein part of the soy hull fibres have been degraded by one or more xylanase(s) into oligosaccharides, comprising 4 % by weight or more of oligosaccharides having from 3 to 30 monomer sugar units, such as 5 % by weight or more, or 10 % by weight or more, of oligosaccharides having from 3 to 30 monomer sugar units (DP3-30). In this context, the oligosaccharides DP 3-30 derived from the dietary fibres do not comprise raffinose, stachyose, and verbascose.

In The feed or food ingredient may be derived from soy hulls, wherein part of the soy hull fibres have been degraded by one or more glucanase(s) into oligosaccharides, comprising 4 % by weight or more of oligosaccharides having from 3 to 30 monomer sugar units, such as 5 % by weight or more, or 10 % by weight or more, of oligosaccharides having from 3 to 30 monomer sugar units (DP3-30). In this context, the oligosaccharides DP 3-30 derived from the dietary fibres do not comprise raffinose, stachyose, and verbascose.

The the feed or food ingredient may be derived from soy hulls, wherein part of the soy hull fibres have been degraded by several of the carbohydrases into oligosaccharides having from 3 to 30 monomer sugar units (DP3-30).

*The method of the invention in its second aspect:*
In the second aspect of the invention, it relates to a method for the production of feed or food ingredient according to the invention, comprising the following steps:
- mixing soy hulls with one or more carbohydrase(s) selected from mannanase(s), pectinase(s), xylanase(s), glucanase(s), and cellulase(s);
- adding yeast to the mixture of soy hulls and carbohydrase(s);
- hydrolysing the mixture under conditions, where the dry matter (DM) content is 55 % by weight or less, in a time period of from 1 to 48 hours, at a temperature from 20 to 60 °C;
- optionally fully inactivating the activity of the one or more carbohydrase(s);
- isolation of the feed or food ingredient.

The skilled person will be able to select a suitable amount of carbohydrase enzyme(s) that fulfils the requirement of the method, depending on the process conditions, the intended result, and the optimal process economy.

In one embodiment of this aspect, the fibre-rich plant biomass may have been disintegrated before being mixed with the one or more carbohydrase(s). The means for disintegration may be physical or chemical, and the skilled person will know whether disintegration is adequate og necessary, and which means are applicable for a specific biomass.

In any embodiment of the inventions in its second aspect, the method includes a step of full inactivating the activity of the one or more carbohydrase(s), conducted after the step of hydrolysing the mixture. The inactivation step secures that the Oligosaccharides DP 3-30 are not degraded into monosaccharides.

In any embodiment of the invention in its second aspect, the dry matter content is 55 % by weight or less, such as 53 % by weight or less, 51 % by weight or less, 50 % by weight or less, 48 % by weight or less, 46 % by weight or less, or 45 % by weight or less.

The reaction time is from 1 to 48 hours, such as 2, 4, 6, 8, 12, 16, 20, 24, 28, 32, 36, 40 or 44 hours.

The reaction temperature is from 20 to 60 °C, such as 25, 30, 32, 35, 37, 40, 45, 50, 55, or 58 °C.

The skilled person will be able to select and adapt reaction time and reaction temperature in proportion to the selected carbohydrase(s) and the amount thereof.

In any embodiment of the invention in its second aspect, yeast, such as live yeast selected among *Saccharomyces cerevisiae* strains, including spent brewer's yeast, baker's yeast, spent distiller's yeast, and spent yeast from wine production, bioethanol yeast, or spent yeast from bioethanol production, and yeast strains fermenting C5 sugars, may be added to the mixture of soy hulls and carbohydrase(s) before the hydrolysing step, e.g. in an amount of from 0.05 to 10 %, such as 0.1%, 0.15%, 0.20 %, 0.25 %, 0.5%, 1%, 1.5%, 2%, 2.5%, 3%, 3.5%, 4%, 4.5, 5%, 6%, 7%, 8% or 9%. The skilled person will be able to select a suitable amount, depending on the process conditions and the intended result.

In any embodiment of the invention in its second aspect, one or more microorganism(s) may be added to the mixture of soy hulls and carbohydrase(s) before the hydrolysing step. Addition of microorganism(s) is suitable for conversion of carbohydrate present in the soy hulls into useful metabolic products, in particular organic acids, such as lactic acid and acetic acid. The skilled person will be able to select a suitable amount, depending on the process conditions and the intended result.

In any embodiment of the invention in its second aspect, lactic acid bacteria, such as *Lactobacillus, Pediococcus, Lactococcus, Enterococcus, Weisella, Streptococcus, and Leuconostoc* may be added to the mixture of soy hulls and carbohydrase(s) before the hydrolysing step. Addition of lactic acid bacteria is suitable for conversion of carbohydrate present in the plant biomass into useful metabolic products, in particular organic acids, such as lactic acid and acetic acid. The skilled person will be able to select a suitable amount, depending on the process conditions and the intended result.

The one or more microorganism(s), may be selected from *Bacillus, Bifidobacterium, Brevibacillus, Propionibacterium, Clostridium, and Geobacillus,* and may be added to the mixture of soy hulls and carbohydrase(s) before the hydrolysing step. The skilled person will be able to select a suitable amount, depending on the process conditions and the intended result.

In any embodiment of the invention in its second aspect, α-galactosidase may be added to the mixture of soy hulls and carbohydrase(s) before the hydrolysing step. Addition of α-galactosidase is suitable for degrading raffinose, stachyose, and verbascose present in soy hulls. The skilled person will be able to select a suitable amount, depending on the process conditions, and the intended result.

*The feed or food product or nutritional supplement of the invention in its third aspect:*
In a third aspect of the invention, it relates to feed or food product or a nutritional supplement containing from 0.5 to 99% by weight of a feed or food ingredient according to the invention.

In any embodiment of the invention in its third aspect, the feed product or nutritional supplement may be suitable for use in a diet for production animals, such as a diet for improving performance in production animals, in particular newborn and young animals, such as piglets, calves, and poultry.

In any embodiment of the invention in its third aspect, the feed product or nutritional supplement may be for use in a diet for production animals, in particular newborn and young animals, such as piglets, calves, and chickens, in need of prebiotic oligosaccharides.

In a 4^{th} aspect of the invention, it relates to use of a feed ingredient according to the invention in a diet for production animals, in particular newborn and young animals, such as piglets, calves, and chickens.

### EXAMPLES

### Material and methods

### Enzymes and enzyme suppliers:

BIO-CAT, Vland Biotech Inc, Strowin, Jinan BestZyme, Winovazyme, Habio, Challenge Group, Biocatalysts Ltd and DSM. Enzymes were supplied between April 2018 to December 2019 and the specific names of the enzymes are seen below.

| | Pectinase | Xylanase | Mannanase | β-glucanase | Cellulase | Blend |
|---|---|---|---|---|---|---|
| BioCat | Pectinase endo 3.5K | Xylanase 150K | | β-glucanase 6K | Cellulase AN 100K | Hemicellulase 400K |
| | | | | | Cellulase 150K | |
| Vland | Pectinase 300K | Xylanase 200K | Neutral mannanase 3K | β-glucanase 50 K | Neutral-cellulase 30K | |
| Strowin | Pectinase 30K | Xylanase 200K | β-mannanase (normal) 50K | β-glucanase 50K | Cellulase 10K | |
| Jinan | Pectinase 30K | Xylanase 600K | β-mannanase 100K | β-glucanase 100K | Cellulase 20K | |
| Winovazyme | | Xylanase 200K | β-mannanase CM 100K | β-glucanase 50K | Cellulase 8 K | |
| DSM | Ronozyme VP | Ronozyme WX | | | | |
| Habio | Pectinase 30K | | β-mannanase 100K | β-glucanase 50K | Cellulase 20K | |
| Challenge | | Xylanase 50K | β-mannanase 50K | β-glucanase 50K | Cellulase 20K | |
| Biocatalysts | Pectinase 62L | Depol 761P | | | Cellulase 13L | Depol 793L |
| | | | | | | Depol 670L |

### Thin Layer Chromatography (TLC method)

A 10% DM suspension of the sample was first prepared and homogenized for 30 sec using a Ultra Thorax instrument at level 2. The sample was stirred at room temperature for 30 min, and centrifugation at 3000 G for 10 min. The supernatant was used directly for TLC.

1.4 µl of samples, and different standard mixes, were added to the TLC plate. After drying, the plate was placed in the chromatography tank for 50-60 min, followed by drying and dipping in developer liquid for 2 second. After drying, TLC patterns became visible after a short time at 150 ⁰C. Chromatography fluid consisted of demineralized water with 46% vol/vol n-butanol and 31% vol/vol pyridine. Developer solution consisted of 2 g diphenylamine, 84 ml acetone, 2 ml aniline and 15 ml 85% phosphoric acid per 100 ml.

### Ion-exchange chromatographic analysis (Dionex)

A 10% DM suspension of the sample was prepared and homogenized for 30 sec using an Ultra Turrax T25 instrument, IKA, at level 2. The sample was stirred at room temperature for 30 min, and centrifugation at 3000 G for 10 min. The supernatant was used for Dionex or Viscotek analysis.

Oligosaccharides and sugars were separated and quantified by high-performance anion exchange chromatography with pulsed amperometric detection (HPAEC-PAD, Dionex). Oligosaccharides were estimated using an external maltodextrin standard. Samples were injected on a CarboPacPA-200 column using 0.4 mL min-1 flow rate, 150mM isocratic NaOH and the following NaOAc gradient profile: 0-5 min: 0-110mM linear gradient, 5-30 min: 110-350mM convex gradient.

### Size exclusion chromatographic analysis (Viscotek)

Samples were examined by size exclusion chromatography (SEC) using a Viscotek System (Malvern, UK) equipped with two columns in series: GS-320 HQ and GS-620 HQ columns (Shodex) attached to a TDA 302 module (Triple detector array) comprising a refractive index detector (RI), a four-bridge viscometer detector (VIS) and a light scattering detector (LS). The LS comprised a right-angle light scattering (RALS) and a low angle light scattering (LALS) that measures the scattered light at 7° and 90° with respect to the incident beam. The instrument was calibrated using pullulan (50 kDa, polydispersity 1.07, Showa Denko) solubilized in MilliQ water (1mg/mL) at 99 °C for 120 min at 1000 rpm. Elution was performed with 50mM ammonium formate (HCO₂NH₄) buffer and 0.5 mL min-1 flow rate. Samples were filtered through a 0.22 µm centrifuge filter and 50 µl sample injected (GPCmax module) into the column and separated at 60 °C. Authentic well-characterised linear maltooligosaccharides were used as external standards. Data were analysed using the OmniSec Software 4.7 (Malvern Instrument, Ltd.).

### Example 1:

### Small scale screening showing the effect of carbohydrases in soy hulls

A total of 40 commercial carbohydrase enzymes covering at least the general groups of mannanases, xylanases, cellulases, β-glucanases, and pectinases, from different suppliers were tested in soy hull biomass. The enzymes were dosed according to enzyme price, adding a dose corresponding to a fixed price per weight of soy hull dry matter, and compared accordingly. Soy hulls were delivered as dry pills, and were roughly disintegrated in a blender. Enzymes and soy hulls were mixed and incubated at a dry matter (DM) content of 45-48% by weight, at 32 °C for 8-44 hours. Approx. 90 g soy hulls were used in this small scale screening. α-galactosidase was added to all samples to remove the oligosaccharides stachyose and raffinose in the soy hull biomass. In some experiments, baker's yeast was added to the reaction mixture. In some experiments, pH was regulated to 4.5 with H₂SO₄.

After hydrolysis of the biomass, its content of Oligosaccharide DP 3-30 (oligosaccharides having 3-30 sugar unit) was determined with either the Dionex or the Viscotek method. The TLC method was also applied for further quantification of the pattern of enzyme-cutting into oligosaccharides by the different enzymes.

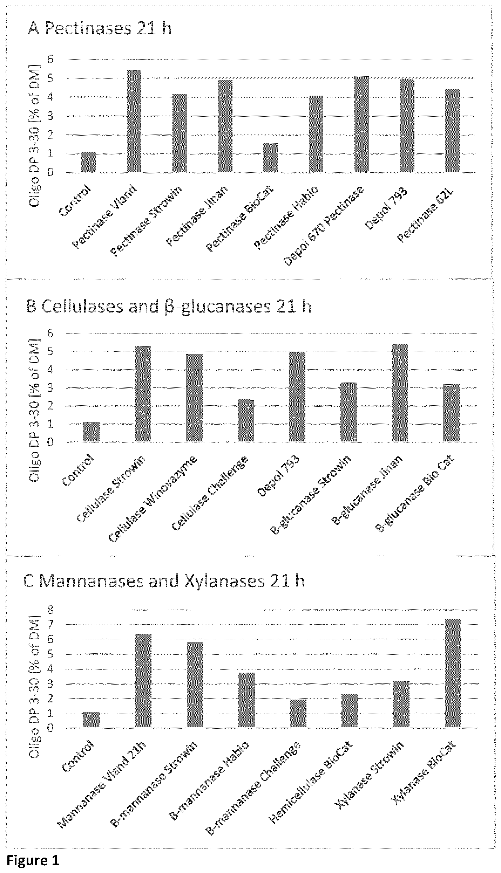

Figure 1 A, B and C show the first screening results after incubation for 21 hours with pH-regulation and analysis of the product with the Dionex method.

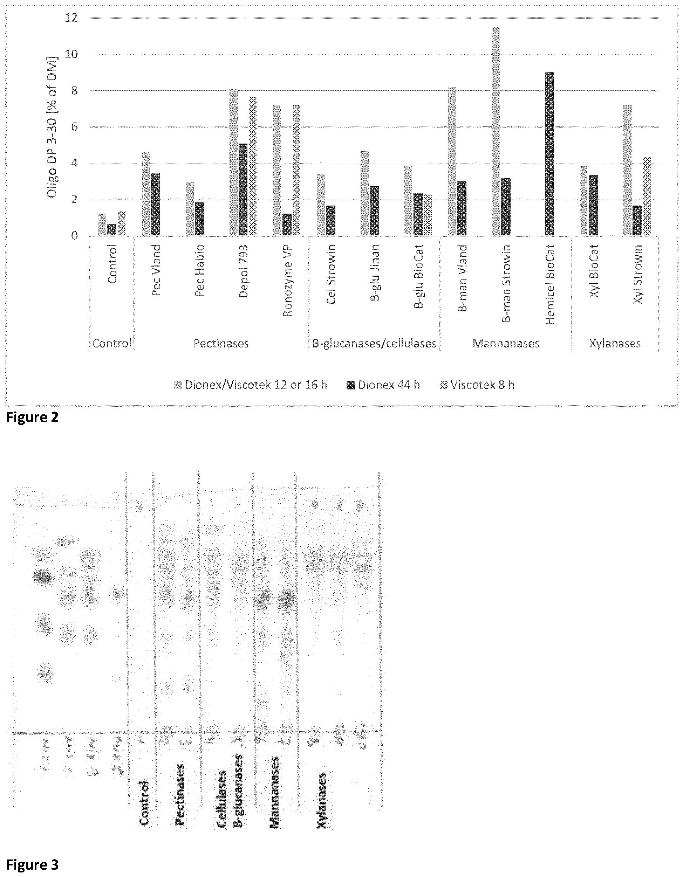

Figure 2 shows the screening results after incubations for 8-44 hours without pH-regulation and analysis of the product with the Dionex and Viscotek method. If more analysis was done with the same enzyme, means were used in the figure.

Figure 3 shows sugar compositions of soy hulls subjected to treatment with different carbohydrase enzymes, clearly showing that different types of enzymes cut differently in the soy hulls, resulting in typical patterns for mannanase, xylanase, pectinase, and β-glucanase/cellulose, respectively. The reference sugar mixtures displayed in lane 1-4 contain as follows reading from the top:
Mix 1: xylose, sucrose, raffinose, stachyose
Mix A: arabinobiose, arabinose, mannobiose, mannotriose
Mix B: xylose, xylobiose, xylotriose, mannobiose, mannotriose
Mix C: galactose, galacturonic acid

The mean maximal obtained oligosaccharide levels obtained in the small scale screening with the different enzyme groups after 16 hours of hydrolysis, are shown as a mean analysis value for the best enzyme below:

| | Oligosaccharides DP 3-30, % by weight of DM |
|---|---|
| Control | 1 |
| B-mannanase | 11 |
| Pectinase | 9 |
| Xylanase | 8 |
| Glucanase/cellulase | 5 |

### Example 2

### Showing the effect of carbohydrases in soy hulls in pilot scale

Three hydrolysis experiments was conducted in a horisontal, slowly turning reactor, applying 450 kg soy hull per pilot experiment and the most effectively working enzymes from the screening example, and baker's yeast and α-galactosidase. Soy hull pills were not disintegrated beforehand, the initial DM and temperature were 48% by weight and 30 °C, and the incubation time was 16 hours.

Viscotek analysis was performed. The content of Oligosaccharide DP 3-30 (oligosaccharides having 3-30 sugar unit) was recalculated to % by weight of the total soy hull biomass.

| | Oligosaccharides DP 3-30, % by weight of DM |
|---|---|
| Best pectinase enzyme | 15.8 |
| Best mannanase enzyme | 22.8 |
| Best xylanase enzyme | 17.0 |

### Example 3

### Showing the effect of incubation time and enzyme dose

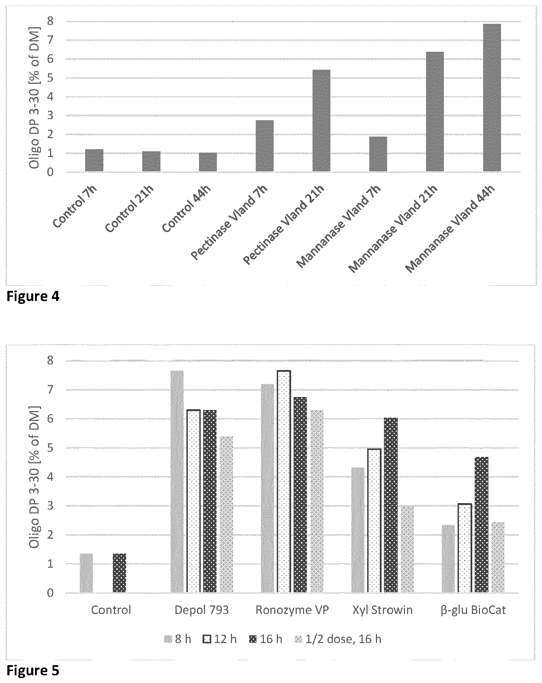

The effect of incubation time was examined using either a hydrolysis condition with pH at 4.5 (figure 4) or an unregulated pH (figure 5), as described in example 1. Figure 5 also shows the effect of including half enzyme dose. As would be expected, the experiments show that the effect of incubation time is enzyme dependent.

### Example 4

Showing oligosaccharide levels and the effect of carbohydrases in other biomasses in comparison with soy hulls

The level of oligosaccharides was tested in a range of different biomasses, in different forms:
a) the raw biomass (control);
b) an incubated biomass without added carbohydrase enzymes (a-galactosidase was added to biomasses containing raffinose, stachyose, and verbascose, in order to remove these oligosaccharides); or
c) the biomass incubated with commercial carbohydrase enzymes.

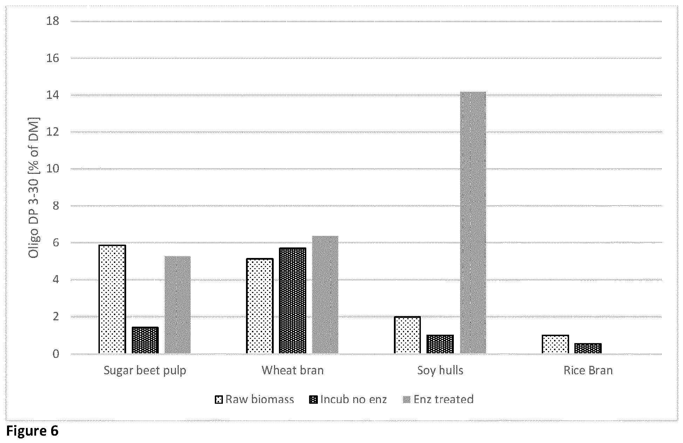

The enzymes were dosed according to price, adding a dose corresponding to a fixed price per weight of biomass dry matter, and compared accordingly. The biomasses sugar beet pulp and soy hulls were delivered as dry pills and were roughly disintegrated in a blender. The other biomasses were used as they were delivered. Enzymes, baker's yeast, and biomass were mixed and incubated at 45 or 48% DM at 32 or 37 °C for 16, 20 or 44 hours. After hydrolysis, the biomass was analysed in the same way as explained in example 1. Results are seen in figure 6. Soy oligosaccharides (raffinose, stachyose and verbascose), if present in the raw biomass, have been subtracted.

Figure 6 shows Dionex or Viscotek quantification of oligosaccharides in the different biomasses, either the raw biomass, an incubated biomass without enzymes, or enzyme treated biomass.

Comparing the release of oligosaccharides on a relative basis (compared to start level in raw material), soy hulls stands out as a biomass where it is possible to release the highest relative amount.

| | Increase factor in oligosaccharides DP 3-30 compared to raw material (exclusive soy oligosaccharides) |
|---|---|
| Sugar beet pulp | 0.9 |
| Wheat bran | 1.2 |
| Soy hulls | 7.2 |

### Example 5

### Showing effect of incubation DM in soy hulls

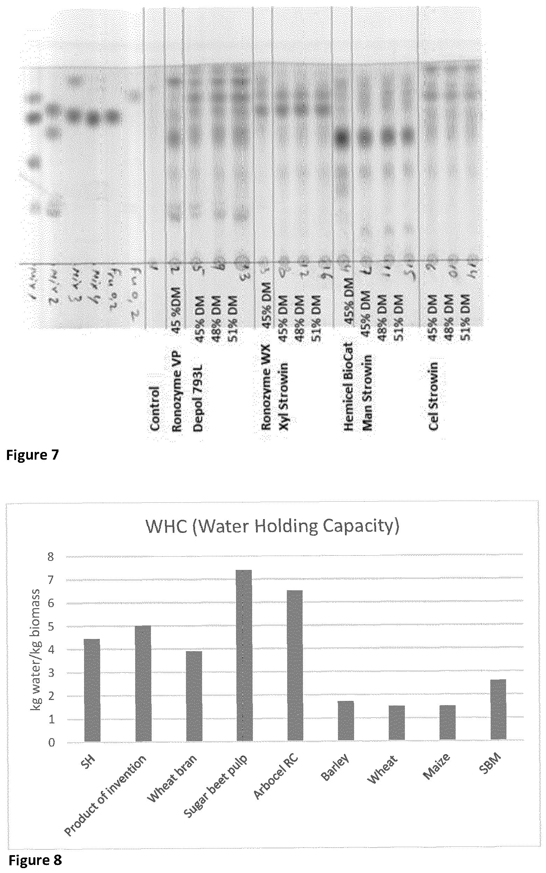

An experiment performed as described in example 1 with non-regulated pH, was performed to test three starting biomass DM levels: 45, 48 and 51% of DM. The results are seen in figure 7, showing a TLC, indicating no effect of increasing DM% from 45% to 51% with Depol 793L (pectinase), xylanase, mannanase and cellulase from Strowin.

### Example 6

A piglet feeding trail was conducted May-August 2018 at a Danish test station: "Skjoldborg teststation, TestGris, SvineRådgivningen, Herning, Denmark". Four diets were tested, using a conventional pig production system and comprising the following:
1. A control with no soy hulls;
2. FaserGold, an extruded soy hull, amount of 2.5% by DM weight;
3. A soy hull incubated with α-galactosidase and baker's yeast for 8 hours, milled and dried, amount of 2% by DM weight;
4. A soy hull incubated with α-galactosidase, Ronozyme VP, and baker's yeast for 7 hours, milled and dried, amount of 2% by DM weight.

The trial ran for 6 weeks following weaning, three separate phases (A, B, and C) of 2 weeks each. The soy hull products were only included during phase A. In phase B and C, piglets were fed the same feed. None of the diets contained antibiotics or therapeutic levels of veterinary ZnO. All diets were fed *ad libitum.* The pigs had permanent access to fresh water. The diets in phase A were wheat (35-38%) and barley (15%) based, using HP300 from Hamlet Protein (19%) as protein source. An equal amount of "premix" was used in each diet (25%), and soy oil was used to equalize energy level in the diets (2.7-3.7%). Wheat and barley were milled, and the diet pelletized. Phase B and C diets were produced by TestGris, following Danish feeding standards.

4111 Danbred crossbred piglets were used, 25±3 days of weaning age, and an average body weight of 6.4 kg. 64 double pens were used. Approx. 2*32 piglets were used in each double pen. The double pens were allocated to one of the 4 diets.

Measurement of the output was done as average daily gain (ADG), feed intake (FI), and feed utilization (FU) (also known as feed conversion rate (FCR). The results as shown in Table 1.

**Table 1 Average daily gain (ADG, g), feed intake (FI) and feed utilisation (FU) in phase A (6-9 kg) phase B (9-15 kg), phase C (15-30 Kg) and the whole test period (A-C) of pigs fed the four experimental diets.^{x}**

| | **Phase** | **Diet 1** | **Diet 2** | **Diet 3** | **Diet 4** | **P-value** |
|---|---|---|---|---|---|---|
| ADG, gld | A | 196 | 200 | 198 | 210 | 0.08 |
| | B | 533 | 531 | 540 | 526 | 0.71 |
| | C | 794 | 792 | 807 | 785 | 0.70 |
| | A-C | 497 | 495 | 507 | 499 | 0.47 |
| FI, g/d | A | 241 | 242 | 239 | 246 | 0.66 |
| | B | 692 | 695 | 695 | 691 | 0.98 |
| | C | 1227 | 1187 | 1224 | 1220 | 0.17 |
| | A-C | 710 | 696 | 709 | 709 | 0.45 |
| FU, kg feed/kg gain | A | 1.25^{b} | 1,23^{ab} | 1,20^{ab} | 1,18^{a} | 0.005 |
| | B | 1.31 | 1.30 | 1.29 | 1.32 | 0.14 |
| | C | 1.56 | 1.55 | 1.52 | 1.57 | 0.62 |
| | A-C | 1.42 | 1.41 | 1.40 | 1.42 | 0.43 |

| | | | | | | |
|---|---|---|---|---|---|---|
| ^{x} Values are LS-means (n=16). ^{ab} LS-Means within rows without a common superscript differ (P<0.05). | | | | | | |

In general, piglets maintained good health during the experiment.

It is concluded that ADG tended to be significantly affected by the dietary treatments in phase A with the highest ADG in the Diet 4 group. The FI was not affected by the dietary treatments, but the FU was significantly higher in the Diet 4 group compared with the Diet 1 group. The different diets fed in phase A did not affect the performance in phase B or C.

### Example 7

Parameters as water holding capacity (WHC) as well as viscosity are important in fibre products. A relatively high water holding capacity in a fibre product ensures satiety feeling in the animal and helps ensure an optimal stool consistency. Viscosity is often an aspect of fibres that give rise to concern, especially in poultry feeding, where a high viscosity is unwanted, as it can slow nutrient absorption and give better room for pathogens to proliferate in the gut. Water holding capacity is measured as the amount of water which can be hold by the biomass against gravity, without release of free water, and the unit is kg water/kg biomass.

### Water holding capacity (WHC)

WHC was measured in a range of dry fibre products, including raw soy hulls, and a finished fibre ingredient according to the present invention, viz. a pectinase treated soy hull as well as barley, wheat, maize, and soy bean meal (SBM). All samples were first disintegrated through a 500 µm sieve. Results are seen in figure 8, and show that the product of the invention have a nicely high WHC, as also characterize many other fibre products.

### Viscosity

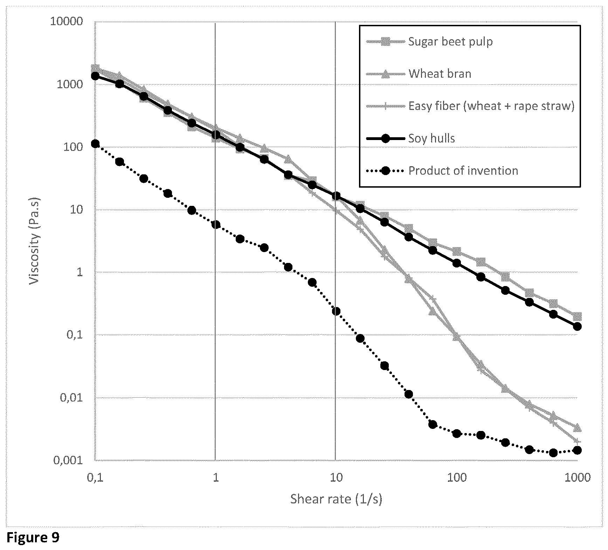

For the viscosity measure the following method was applied: All samples was milled through a 500 µm sieve. 1g sample and 20 mL 0.05M phosphate buffer (pH 7) were incubated at 40 C° for 0.5h. After that, samples were centrifuged at 500 rpm (31 G) for 10min, in a Allegar X-22R centrifuge, Beckman Coulter, before the rheology analysis. The samples were analysed on a Discovery HR-3 Rheometer (TA Instruments). A finished fibre ingredient according to the present invention, viz. a pectinase treated soy hull is compared to raw soy hulls, sugar beet pulp, wheat bran and Easy Fibre (a blend of rape straw and wheat straw). Results are seen in figure 9 and show that compared to raw soy hulls, wheat bran, sugar beet pulp and Easy Fibre, the product of the invention has reduced viscosity. Shear rates relevant in the gut are approximately between 1 and 10 s ⁻¹ is indicated in the figure.

### REFERENCES

Aachary & Prapulla (2011). Xylooligosaccharides (XOS) as an Emerging Prebiotic: Microbial Synthesis, Utilization, Structural Characterization, Bioactive Properties, and Applications. Comprehensive Reviews in Food Science and Food Safety Vol. 10, 2011
Babber et al 2015. Pectic oligosaccharides from agricultural by-products: production, characterization and health benefits. Crit Rev Biotechnol: 1-13
Dotsenko et al (2017). Enzymatic production of wheat and ryegrass derived xylooligosaccharides and evaluation of their in vitro effect on pig gut microbiota. Biomass Conversion and Biorefinery
Chung et al (2017). Prebiotic potential of pectin and pectic oligosaccharides to promote antiinflammatory commensal bacteria in the human colon. FEMS Microbiology Ecology, 93
Corrigan et al (2015). Phylogenetic and Functional Alterations in Bacterial Community Compositions in Broiler Ceca as a Result of Mannan Oligosaccharide Supplementation. Applied and Environmental Microbiology May 2015 Volume 81 Number 10
Englyst HN, Quigley ME and Hudson GJ (1994) Analyst, 119, 1497-1509
Gaggia et al (2010). Probiotics and prebiotics in animal feeding for safe food production. International Journal of Food Microbiology 141 pp15-28
Ghoddusi et al (2007). In vitro study on gas generation and prebiotic effects of some carbohydrates and their mixtures. Anaerobe 13 pp 193-199
Kiarie et al (2013). The role of added feed enzymes in promoting gut health in swine and poultry. Nutrition Research Reviews 26, 71-88
Kim et al (2011). Effect of dietary prebiotic supplementation on the performance, intestinal microflora, and immune response of broilers. Poultry Science 90 :75-82
Kim et al (2019). Potential for Prebiotics as Feed Additives to Limit Foodborne Campylobacter Establishment in the Poultry Gastrointestinal Tract. Frontiers in Microbiology. Volume 10, Article 91
Kurakake et al (2006). Production of Galacto-manno-oligosaccharides from Guar Gum by β-Mannanase from Penicillium oxalicum SO. J. Agric. Food Chem. 54, 7885-7889
Liu et al (2018). Effect of probiotics and xylo-oligosaccharide supplementation on nutrient digestibility, intestinal health and noxious gas emission in weanling pigs. Asian-Australas J Anim Sci Vol. 31, No. 10 pp 1660-1669
Markowiak & Slizewska (2018). The role of probiotics, prebiotics and synbiotics in animal nutrition. Gut Pathog 10:21
Maribo (2005). Tilsaetningsstoffer til svin. Landsudvalget for svin.
Meyer et al (2015). Biotechnological production of oligosaccharides - application in the food industry. Chapter 2 in: Food production and industry https://www.intechopen.com/books/food-production-and-industry
Miguez et al (2016). Pectic oligosaccharides and other emerging prebiotics. Chapter 15 in: Probiotics and prebiotics in human nutrition and health https://www.intechopen.com/books/probiotics-and-prebiotics-in-human-nutrition-and-health
Molist et al (2009) Effects of the insoluble and soluble dietary fibre on the physicochemical properties of digesta and the microbial activity in early weaned piglets. Animal Feed Science and Technology 149 pp 346-353
Moura et al (2018). In vitro fermentation of selected xylo-oligosaccharides by piglet intestinal microbiota. Food Science and Technology 41 pp 1952-1961
Nielsen et al (2014). Diets high in resistant starch and arabinoxylan modulate digestion processes and SCFA pool size in the large intestine and faecal microbial composition in pigs. British Journal of Nutrition 112, 1837-1849
Roberfroid et al (2010). Prebiotic effect: metabolic and health effects https://www.cambridge.org/core/services/aop-cambridge-core/content/view/F644C98393E2B3EB64A562854115D368/50007114510003363a.pdf/pre biotic effects metabolic and health benefits.pdf
Pourabeden & Zhao (2015). MINIREVIEW - Environmental Microbiology. Prebiotics and gut microbiota in chickens FEMS Microbiology Letters, 362
Probert el at (2004). Polydextrose, Lactitol, and Fructo-Oligosaccharide Fermentation by Colonic Bacteria in a Three-Stage Continuous Culture System. Applied and Environmental Microbiology vol 70 no 8 pp 4505-45
Scapini et al (2018). Nutritional Evaluation of Soybean Hulls with or without β-Mannanase Supplement on Performance, Intestinal Morphometric and Carcass Yield of Broilers Chickens. Brazilian Journal of Poultry Science
Smiricky-Tjardes et al (2003). In vitro fermentation characteristics of selected oligosaccharides by swine fecal microflora. J. Anim. Sci. 2003. 81:2505-2514
Strube et al (2015). In Situ Prebiotics for Weaning Piglets: In Vitro Production and Fermentation of Potato Galacto-Rhamnogalacturonan. Applied and Environmental Microbiology vol 81 no 5 pp 1668-78
Torres et al (2010). Galacto-oligosaccharides. Production, properties, applications and significance as prebiotics. Comprehensive reviews in food science and food safety. https://onlinelibrary.wiley.com/doi/pdf/10.1111/1.1541-4337.2010.00119.x
Zivkovic et al 2011. PREBIOTICS IN NUTRITION OF SOWS AND PIGLETS. Biotechnology in Animal Husbandry 27 (3), p 547-559

## Claims

1. A feed or food ingredient derived from soy hulls, wherein the feed or food ingredient comprises dietary fibres from soy hulls in the form of soluble and insoluble polysaccharides, and wherein part of the dietary fibres from the soy hulls have been degraded by one or more carbohydrase(s) selected from mannanase(s), pectinase(s), xylanase(s), glucanase(s), and cellulase(s) into oligosaccharides having from 3 to 30 monomer sugar units (Oligosaccharides DP 3-30) to provide the feed or food ingredient comprising 4 % by weight or more of said Oligosaccharides DP 3-30, calculated as not including the soy oligosaccharides raffinose, stachyose, and verbascose, wherein the feed or food ingredient further comprises yeast, and with the proviso that the feed or food ingredient does not comprise more than 5 % by weight monosaccharides.

2. Feed or food ingredient according to claim 1, wherein part of the dietary fibres from soy hulls have been degraded by the one or more carbohydrase(s) into oligosaccharides (Oligosaccharides DP 3-30) providing prebiotic effect.

3. Feed or food ingredient according to any of the previous claims comprising 5 % by weight or more of said oligosaccharides having from 3 to 30 monomer sugar units (Oligosaccharides DP 3-30), such as 6 % by weight or more, 7 % by weight or more, 8 % by weight or more, 9 % by weight or more, 10 % by weight or more, 12 % by weight or more, 15 % by weight or more, or 20% by weight or more, of the oligosaccharides having from 3 to 30 monomer sugar units (Oligosaccharides DP 3-30).

4. Feed or food ingredient according to any of the previous claims, comprising yeast selected among *Saccharomyces cerevisiae* strains, including spent brewer's yeast and spent distiller's yeast and spent yeast from wine production, bioethanol yeast, or spent yeast from bioethanol production, baker's yeast, and yeast strains fermenting C5 sugars.

5. Feed or food ingredient according to any of the previous claims, comprising yeast in an amount of from 0.05 to 10%, such as 0.10%, 0.15%, 0.20%, 0.25%, 0.5%, 1%, 1.5%, 2%, 2.5%, 3%, 3.5%, 4%, 4.5, 5%, 6%, 7%, 8% or 9%.

6. Feed or food ingredient according to any of the previous claims, further comprising that oligosaccharides raffinose, stachyose, and verbascose present in the soy hulls have been fully or partly degraded.

7. Feed or food ingredient according to any of the previous claims, further comprising one or more microorganisms and/or metabolic products of carbohydrate degradation of the soy hulls by the one or more microorganisms.

8. Feed or food ingredient according to claim 7, further comprising lactic acid bacteria and/or metabolic products of carbohydrate degradation of the soy hulls by lactic acid bacteria.

9. A method for the production of feed or food ingredient according to any of the previous claims, comprising the following steps:
• mixing soy hulls with one or more carbohydrase(s) selected from mannanase(s), pectinase(s), xylanase(s), glucanase(s), and cellulase(s);
• adding yeast to the mixture of soy hulls and carbohydrase(s);
• hydrolysing the mixture under conditions, where the DM content is 55 % by weight or less, in a time period of from 1 to 48 hours, at a temperature from 20 to 60 °C;
• optionally fully inactivating the activity of the one or more carbohydrase(s);
• isolation of the feed or food ingredient.

10. Method according to claim 9, wherein the soy hulls has been disintegrated before being mixed with the one or more carbohydrase(s).

11. Method according to any of the claims 9 or 10, comprising that yeast selected among *Saccharomyces cerevisiae* strains, including spent brewer's yeast and spent distiller's yeast and spent yeast from wine production, bioethanol yeast, or spent yeast from bioethanol production, baker's yeast, and yeast strains fermenting C5 sugars, is added to the mixture of soy hulls and carbohydrase(s) before the hydrolysing step.

12. Method according to any of the claims 9 to 11, further comprising that one or more microorganism(s) is added to the mixture of soy hulls and carbohydrase(s) before the hydrolysing step.

13. Method according to any of the claim 12, further comprising that lactic acid bacteria is added to the mixture of soy hulls and carbohydrase(s) before the hydrolysing step.

14. Method according to any of the claims 9 to 13, further comprising that α-galactosidase is added to the mixture of soy hulls and carbohydrase(s) before the hydrolysing step.

15. A feed or food product or a nutritional supplement containing from 0.5 to 99% by weight of a feed or food ingredient according to any one of the claims 1 to 8.

16. A feed product or a nutritional supplement according to claim 15 suitable for use in a diet for production animals, such as a diet for improving performance.

17. A feed product according to any of the claims 15 to 16 suitable for use in a diet for production animals, preferably newborn and young animals, such as piglets, calves, and chickens, in need of prebiotic oligosaccharides.

18. Use of a feed ingredient according to any of the claims 1 to 8 in a diet for product animals, preferably newborn and young animals, such as piglets, calves, and chickens.

## Patentansprüche

1. Aus Sojahülsen gewonnener Futter- oder Lebensmittelbestandteil, wobei der Futter- oder Lebensmittelbestandteil Ballaststoffe aus Sojahülsen in der Form löslicher und unlöslicher Polysacharide umfasst und wobei ein Teil der Ballaststoffe aus den Sojahülsen durch eine oder mehrere Carbohydrasen, die aus Mannanase(n), Pektinase(n), Xylanase(n), Glucanase(n) und Cellulase(n) ausgewählt sind, zu Oligosachariden, die von 3 bis 30 monomere Zuckereinheiten (Oligosacharide DP 3-30) aufweisen, abgebaut worden ist, um den Futter- oder Lebensmittelbestandteil bereitzustellen, der 4 oder mehr Gewichts-% der Oligosacharide DP 3-30, berechnet als nicht einschließlich der Sojaoligosacharide Raffinose, Stachyose und Verbascose, umfasst, wobei der Futter- oder Lebensmittelbestandteil ferner Hefe umfasst und mit der Maßgabe, dass der Futter- oder Lebensmittelbestandteil nicht mehr als 5 Gewichts-% Monosacharide umfasst.

2. Futter- oder Lebensmittelbestandteil nach Anspruch 1, wobei ein Teil der Ballaststoffe aus Sojahülsen durch die eine oder die mehreren Carbohydrasen zu Oligosachariden (Oligosachariden DP 3-30), die eine präbiotische Wirkung bereitstellen, abgebaut worden ist.

3. Futter- oder Lebensmittelbestandteil nach einem der vorhergehenden Ansprüche, umfassend 5 oder mehr Gewichts-% der Oligosacharide, die von 3 bis 30 monomere Zuckereinheiten (Oligosacharide DP 3-30) aufweisen, wie etwa 6 oder mehr Gewichts-%, 7 oder mehr Gewichts-%, 8 oder mehr Gewichts-%, 9 oder mehr Gewichts-%, 10 oder mehr Gewichts-%, 12 oder mehr Gewichts-%, 15 oder mehr Gewichts-% oder 20 oder mehr Gewichts-% der Oligosacharide, die von 3 bis 30 monomere Zuckereinheiten (Oligosacharide DP 3-30) aufweisen.

4. Futter- oder Lebensmittelbestandteil nach einem der vorhergehenden Ansprüche, umfassend Hefe, die aus Stämmen von *Saccharomyces cerevisiae,* einschließlich verbrauchter Brauerhefe und verbrauchter Destillateurhefe und verbrauchter Hefe aus Weinherstellung, Bioethanolhefe oder verbrauchter Hefe aus Bioethanolherstellung, Bäckerhefe und C5-zuckervergärender Hefestämme, ausgewählt ist.

5. Futter- oder Lebensmittelbestandteil nach einem der vorhergehenden Ansprüche, umfassend Hefe in einer Menge von 0,05 bis 10 %, wie etwa 0,10 %, 0,15 %, 0,20 %, 0,25 %, 0,5 %, 1 %, 1,5 %, 2 %, 2,5 %, 3 %, 3,5 %, 4 %, 4,5 %, 5 %, 6 %, 7 %, 8 % oder 9 %.

6. Futter- oder Lebensmittelbestandteil nach einem der vorhergehenden Ansprüche, ferner umfassend, dass Oligosacharide Raffinose, Stachyose und Verbascose, die in den Sojahülsen vorhanden sind, sämtlich oder teilweise abgebaut worden sind.

7. Futter- oder Lebensmittelbestandteil nach einem der vorhergehenden Ansprüche, ferner umfassend einen/ein oder mehrere Mikroorganismen und/oder Stoffwechselprodukte von Kohlenhydratabbau der Sojahülsen durch den einen oder die mehreren Mikroorganismen.

8. Futter- oder Lebensmittelbestandteil nach Anspruch 7, ferner umfassend Milchsäurebakterien und/oder Stoffwechselprodukte von Kohlenhydratabbau der Sojahülsen durch Milchsäurebakterien.

9. Verfahren zur Herstellung eines Futter- oder Lebensmittelbestandteils nach einem der vorhergehenden Ansprüche, umfassend die folgenden Schritte:
• Mischen von Sojahülsen mit einer oder mehreren Carbohydrasen, die aus Mannanase(n), Pektinase(n), Xylanase(n), Glucanase(n) und Cellulase(n) ausgewählt sind;
• Hinzugeben von Hefe zu dem Gemisch von Sojahülsen und Carbohydrase(n);
• Hydrolysieren des Gemisches unter Bedingungen, bei denen der Trockenmassegehalt 55 oder weniger Gewichts-% beträgt, in einem Zeitraum von 1 bis 48 Stunden bei einer Temperatur von 20 bis 60 °C;
• optional vollständiges Inaktivieren der Aktivität der einen oder der mehreren Carbohydrasen;
• Isolieren des Futter- oder Lebensmittelbestandteils.

10. Verfahren nach Anspruch 9, wobei die Sojahülsen zersetzt worden sind, bevor sie mit der einen oder den mehreren Carbohydrasen gemischt werden.

11. Verfahren nach einem der Ansprüche 9 oder 10, umfassend, dass Hefe, die aus Stämmen von *Saccharomyces cerevisiae,* einschließlich verbrauchter Brauerhefe und verbrauchter Destillateurhefe und verbrauchter Hefe aus Weinherstellung, Bioethanolhefe oder verbrauchter Hefe aus Bioethanolherstellung, Bäckerhefe und C5-zuckervergärender Hefestämme, ausgewählt ist, vor dem Schritt des Hydrolysierens zu dem Gemisch von Sojahülsen und Carbohydrase(n) hinzugegeben wird.

12. Verfahren nach einem der Ansprüche 9 bis 11, ferner umfassend, dass ein oder mehrere Mikroorganismen vor dem Schritt des Hydrolysierens zu dem Gemisch von Sojahülsen und Carbohydrase(n) hinzugegeben wird/werden.

13. Verfahren nach Anspruch 12, ferner umfassend, dass Milchsäurebakterien vor dem Schritt des Hydrolysierens zu dem Gemisch von Sojahülsen und Carbohydrase(n) hinzugegeben werden.

14. Verfahren nach einem der Ansprüche 9 bis 13, ferner umfassend, dass α-Galactosidase vor dem Schritt des Hydrolysierens zu dem Gemisch von Sojahülsen und Carbohydrase(n) hinzugegeben wird.

15. Futter- oder Lebensmittelprodukt oder Nahrungsergänzungsmittel, enthaltend von 0,5 bis 99 Gewichts-% eines Futter- oder Lebensmittelbestandteils nach einem der Ansprüche 1 bis 8.

16. Futterprodukt oder Nahrungsergänzungsmittel nach Anspruch 15, das zur Verwendung in einer Ernährung für Nutztiere, wie etwa einer Ernährung zur Leistungsverbesserung, geeignet ist.

17. Futterprodukt nach einem der Ansprüche 15 bis 16, das zur Verwendung in einer Ernährung für Nutztiere, bevorzugt neugeborene und junge Tiere, wie etwa Ferkel, Kälber und Hühner, die präbiotischer Oligosacharide bedürfen, geeignet ist.

18. Verwendung eines Futterbestandteils nach einem der Ansprüche 1 bis 8 in einer Ernährung für Nutztiere, bevorzugt neugeborene und junge Tiere, wie etwa Ferkel, Kälber und Hühner.

## Revendications

1. Alimentation ou ingrédient alimentaire dérivé de coques de soja, dans lequel l'aliment ou l'ingrédient alimentaire comprend des fibres alimentaires provenant de coques de soja sous la forme de polysaccharides solubles et insolubles, et dans lequel une partie des fibres alimentaires provenant de coques de soja a été dégradée par une ou plusieurs carbohydrases sélectionnées parmi une ou des mannanase(s), pectinase(s), xylanase(s), glucanase(s) et cellulase(s) en oligosaccharides ayant de 3 à 30 motifs de sucre monomères (oligosaccharides DP 3-30) pour fournir l'aliment ou l'ingrédient alimentaire comprenant 4 % en poids ou plus desdits oligosaccharides DP 3-30, calculés comme n'incluant pas les oligosaccharides de soja raffinose, stachyose et verbascose, ou dans lequel l'aliment ou l'ingrédient alimentaire comprend en outre de la levure, et à condition que l'aliment ou l'ingrédient alimentaire ne comprenne pas plus de 5 % en poids de monosaccharides.

2. Alimentation ou ingrédient alimentaire selon la revendication 1, ou dans lequel une partie des fibres alimentaires provenant de coques de soja a été dégradée par les une ou plusieurs carbohydrase(s) en oligosaccharides (oligosaccharides DP 3-30) fournissant un effet prébiotique.

3. Alimentation ou ingrédient alimentaire selon l'une quelconque des revendications précédentes comprenant 5 % en poids ou plus desdits oligosaccharides ayant de 3 à 30 unités de sucre monomères (oligosaccharides DP 3-30), comme 6 % en poids ou plus, 7 % en poids ou plus, 8 % en poids ou plus, 9 % en poids ou plus, 10 % en poids ou plus, 12 % en poids ou plus, 15 % en poids ou plus, ou 20 % en poids ou plus, des oligosaccharides ayant de 3 à 30 motifs de sucre monomères (oligosaccharides DP 3-30).

4. Alimentation ou ingrédient alimentaire selon l'une quelconque des revendications précédentes, comprenant une levure sélectionnée parmi des souches de *Saccharomyces cerevisiae,* y compris la levure de bière épuisée et la levure de distillerie épuisée et la levure épuisée provenant de la production de vin, la levure de bioéthanol, ou la levure épuisée provenant de la production de bioéthanol, la levure de boulanger et des souches de levure fermentant des sucres C5.

5. Alimentation ou ingrédient alimentaire selon l'une quelconque des revendications précédentes, comprenant de la levure en une quantité de 0,05 à 10 %, telle que 0,10 %, 0,15 %, 0,20 %, 0,25 %, 0,5 %, 1 %, 15 %, 2 %, 2,5 %, 3 %, 3,5 %, 4 %, 4,5, 5 %, 6 %, 7 %, 8 % ou 9 %.

6. Alimentation ou ingrédient alimentaire selon l'une quelconque des revendications précédentes, comprenant en outre le fait que les oligosaccharides raffinose, stachyose et verbascose présents dans les coques de soja ont été entièrement ou partiellement dégradés.

7. Alimentation ou ingrédient alimentaire selon l'une quelconque des revendications précédentes, comprenant en outre un ou plusieurs micro-organismes et/ou produits métaboliques de dégradation des glucides des coques de soja par les un ou plusieurs micro-organismes.

8. Alimentation ou ingrédient alimentaire selon la revendication 7, comprenant en outre des bactéries d'acide lactique et/ou des produits métaboliques de dégradation des glucides des coques de soja par des bactéries d'acide lactique.

9. Procédé de production d'alimentation ou d'ingrédient alimentaire selon l'une quelconque des revendications précédentes, comprenant les étapes suivantes :
• le mélange de coques de soja avec une ou plusieurs carbohydrase(s) sélectionnée(s) parmi une ou des mannanase(s), pectinase(s), xylanase(s), glucanase(s) et cellulase(s) ;
• l'ajout de levure au mélange de coques de soja et de carbohydrase(s) ;
• l'hydrolyse du mélange dans des conditions dans lesquelles la teneur en MS est de 55 % en poids ou moins, sur une période de temps de 1 à 48 heures, à une température de 20 à 60 °C ;
• éventuellement l'inactivation totale de l'activité des une ou plusieurs carbohydrase(s) ;
• l'isolement de l'alimentation ou de l'ingrédient alimentaire.

10. Procédé selon la revendication 9, dans lequel les coques de soja ont été désintégrées avant d'être mélangées avec les une ou plusieurs carbohydrase(s).

11. Procédé selon l'une quelconque des revendications 9 ou 10, comprenant le fait qu'une levure sélectionnée parmi des souches de *Saccharomyces cerevisiae,* y compris la levure de bière épuisée et la levure de distillerie épuisée et la levure épuisée provenant de la production de vin, la levure de bioéthanol, ou la levure épuisée provenant de la production de bioéthanol, la levure de boulanger et des souches de levure fermentant des sucres C5, est ajoutée au mélange de coques de soja et de carbohydrase(s) avant l'étape d'hydrolyse.

12. Procédé selon l'une quelconque des revendications 9 à 11, comprenant en outre le fait qu'un ou plusieurs micro-organismes sont ajoutés au mélange de coques de soja et de carbohydrase(s) avant l'étape d'hydrolyse.

13. Procédé selon la revendication 12, comprenant en outre le fait que des bactéries d'acide lactique sont ajoutées au mélange de coques de soja et de carbohydrase(s) avant l'étape d'hydrolyse.

14. Procédé selon l'une quelconque des revendications 9 à 13, comprenant en outre le fait que de l'α-galactosidase est ajoutée au mélange de coques de soja et de carbohydrase(s) avant l'étape d'hydrolyse.

15. Alimentation ou produit alimentaire ou complément nutritionnel contenant de 0,5 à 99 % en poids d'une alimentation ou d'un ingrédient alimentaire selon l'une quelconque des revendications 1 à 8.

16. Produit alimentaire ou complément nutritionnel selon la revendication 15, approprié pour être utilisé dans un régime alimentaire pour animaux de production, tel qu'un régime destiné à améliorer les performances.

17. Produit alimentaire selon l'une quelconque des revendications 15 et 16, approprié pour être utilisé dans un régime alimentaire pour animaux de production, de préférence des animaux nouveau-nés et jeunes, tels que des porcelets, des veaux et des poulets, ayant besoin d'oligosaccharides prébiotiques.

18. Utilisation d'un ingrédient alimentaire selon l'une quelconque des revendications 1 à 8 dans un régime alimentaire pour animaux de produits, de préférence des animaux nouveau-nés et jeunes, tels que des porcelets, des veaux et des poulets.
